# EUROPEAN PATENT APPLICATION

(11) **EP 2 385 372 A1**
(43) Date of publication of application: **09.11.2011**
(21) Application number: 11170804.6
(22) Date of filing: 21.06.2011
(51) Int. Cl.: G01N 33/68, G01N 33/92

(54) **Kidney disease in normal and abnormal pregnancy**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention relates to the field of diagnostic measures. Specifically, it relates to a method for diagnosing kidney damage during or after pregnancy. The method comprises the steps of determining the amount of adiponectin in a sample of a subject and comparing the determined amount to a reference amount for adiponectin. Said reference amount shall be derived from a pregnant subject of or from a subject who has delivered a child. Further encompassed are a diagnostic device and a kit for carrying out the aforementioned method.

## Description

The present invention relates to the field of diagnostic measures. Specifically, it relates to a method for diagnosing kidney damage during or after pregnancy. The method comprises the steps of determining the amount of adiponectin in a sample of a subject and comparing the determined amount to a reference amount for adiponectin. Said reference amount shall be derived from a pregnant subject of or from a subject who has delivered a child. Further encompassed are a diagnostic device and a kit for carrying out the aforementioned method.

Pregnancy may be complicated in different ways, it is on one hand associated with pregnancy related mortality of the pregnant woman. Maternal mortaliy at a rate of 14,5 per 100.000 live births, is more frequent in pregnant women above the age of 39 years and may be caused by hemorrage, thrombotic pulmonary embolism, infections, cardiomyopathy and cardiovascular and non-cardiovascular conditions as well as hypertensive disorders among which pre-eclampsia is the most frequent.(Berg 2010, Obstretics and Gynecology: 116: 1302 - 1309).

Chronic kidney disease is often clinically and biochemically silent until renal impairment is advanced (Williams D. et al BMJ 2008: 336: 211-5.). In pregnancy underlying or not underlying kidney disease may be affected by recurrent urinary tract infection or preeclampsia, Williams D. et al, see above). Pregnant woman with chronic renal disease adapt poorly to a gestational increase in renal blood flow. This may accelerate the decline in kidney function and lead to a poor pregnancy outcome.

Kidney damage usually is diagnosed via the determination of the glomerular filtration rate (rate) or by the determination of serum creatinine. However, physiological changes during pregnancy complicate the assessment of the kidney in pregnancy. in the course of pregnancy physiological increases in renal blood flow of more than 50 % or more occur.(Davidson J. et al in De Swiet M et al, in Medical disorders in Medical practice 3rd ed. Oxford: Blackwell Science, 1995: 226 - 305). Moreover, gestational hyperfiltration is accompanied by a relative decrease in concentrations of serum creatinine and urea, so values considered normal in the non-pregnant state may be abnormal in pregnancy. (Williams D. et al BMJ). Therefore, the serum creatinine levels and the GFR are less reliable indicator for kidney damage during pregnancy.

Urinary adiponectin has been proposed as a marker for vascular damage in type 2 diabetes (see von Eynatten, 2009, Diabetes, 58: 2093 to 2099). No information, however, is available whether adiponectin is a marker for kidney disease during or after pregnancy.

There is a need for additional markers in the assessment of kidney disease in pregnancy.

Thus, there is an urgent need to refine the diagnosis of kidney damage in pregnant women.

The technical problem underlying the present invention can be seen as the provision of means and methods for reliably and efficiently diagnosing kidney damage in a pregnant subject. The technical problem is solved by the embodiments characterized in the claims and herein below.

Accordingly, the present invention relates to a method for diagnosing kidney damage in a subject during or after pregnancy, comprising the steps of
a. determining the amount of adiponectin in a sample from said subject, and
b. comparing the amount of adiponectin in said sample from a subject with a reference amount for adiponectin, said reference amount being derived from a subject during or after pregnancy, whereby kidney damage is to be diagnosed.

Preferably, it is diagnosed whether said subject suffers from kidney damage, or not, by carrying out the further step of c) diagnosing whether said subject suffers from kidney damage, or not, based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b). More preferably, the method is carried out entirely in an automated manner. In such a case, the diagnostic result which is established in step b) is generated in a suitable output format so that it can be used as an aid for establishing the final clinical diagnosis by, e.g., a medical practitioner.

The term "diagnosing" as used herein means assessing whether a subject as referred to in accordance with the method of the present invention suffers from kidney damage, or whether said subject does not suffer from kidney damage. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Thus, the method of the present invention, however, at least provides an aid for establishing a final clinical diagnosis. Whether a portion is statistically significant, can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The method of the present invention shall allow for the diagnosis of kidney damage in the subject to be tested. In the context of the present invention, the term "kidney damage", preferably, refers to glomerular damage, i.e. to damage of the glomeruli of the kidney. Said damage is, preferably, caused by epithelial injury in glomerular cells. The present invention preferably refers to chronic glomerular damage.

Many diseases affect kidney function by damaging the glomeruli. Diseases including glomerular damage include many conditions with a variety of genetic and environmental causes, but they fall into two major categories: i) glomerulonephritis and ii) glomerulosclerosis. In glomerulonephritis, the membrane tissue in the kidney that serves as a filter is inflammated. In glomerulosclerosis, blood vessels within the kidney are scarred or hardened. In the context of the present invention, the glomerular damage is, preferably, glomerulosclerosis.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma, or serum and, most preferably, urine. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

If the subject is tested during pregnancy, i.e. if the sample is obtained during pregancy the following applies: Preferably, the sample is derived from the subject being in any one of week of gestation 9 to 38. Even more preferably, the sample is derived from a subject being in any one of week of gestation 28 to 37. Most preferably, the subject is in week 38 of gestation or later (i.e. up to delivery of the child).

If the subject is tested after delivery of the child, i.e. if the sample is obtained after pregnancy, the following applies: Preferably, the sample is obtained not later than one year after delivery. More preferably, the sample is obtained not later than six months, or not later than three months after delivery. Even more preferably, the sample is obtained not later than six weeks, or not later than three after delivery. Most preferably, the sample is obtained not later than 1 week after delivery.

In accordance with the methods of the present invention, the subject shall be tested during or after pregnancy. Accordingly, the subject to be tested shall be a woman.

If the subject to be tested is a pregnant subject, the subject is, preferably, in any one of week of gestation 9 to 38. More preferably, the subject is in any one of week of gestation 28 to 37. Most preferably, the subject is in week 38 of gestation or later.

However, it is also contemplated that the subject is tested after pregnancy, in particular after delivery of child. In this case, the sample is obtained not later than one year after delivery. More preferably, the sample is obtained not later than six months, or not later than three months after delivery. Even more preferably, the sample is obtained not later than six weeks, or not later than three after delivery. Most preferably, the sample is obtained not later than 1 week after delivery.

In a preferred embodiment of the present invention, the subject to be tested, preferably, suffers from diabetes mellitus or pre-eclampsia. Diabetes mellitus includes preferably, type 2 diabetes mellitus, and, more preferably, type 1 diabetes mellitus. Preferably, however, the subject shall not suffer from gestational diabetes. It is further envisaged that the subject to be tested does not suffer from urinary tract infection. Moreover, the subject, preferably, does not have impaired renal function. How to assess whether a subject exhibits impaired renal function is well known in the art. Renal disorders can be diagnosed by any means known and deemed appropriate. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. The GFR was originally estimated (the GFR can never be determined, all calculations derived from formulas such as the Cockgroft Gault formula of the MDRD formula deliver only estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 ml/min. Thus, it is particularly contemplated that the GFR of the subject as referred to herein is within this range. Moreover, the subject as referred to in the context of the method present invention, preferably, has a blood creatinine level (in particular a serum creatinine level) of lower than 0.9 mg/dl, more preferably of lower than 1.1 mg/dl and most preferably of lower than 1.3 mg/dl.

Adiponectin is a polypeptide (one of several known adipocytokines) secreted by the adipocyte. In the art, adiponectin is frequently also referred to as Acrp30 and apM1. Adiponectin has recently been shown to have various activities such as anti-inflammatory, antiatherogenic, preventive for metabolic syndrome, and insulin sensitizing activities. Adiponectin is encoded by a single gene, and has 244 amino acids, the molecular weight is approximately 30 kilodaltons. The mature human adiponectin protein encompasses amino acids 19 to 244 of full-length adiponectin. A globular domain is thought to encompass amino acids 107 - 244 of full-length adiponectin. The sequence of the adiponectin polypeptide is well known in the art, and, e.g., disclosed in WO2008/084003.

Adiponectin associates itself into larger structures. Three adiponectin polypeptides bind together and form a homotrimer. These trimers bind together to form hexamers or dodecamers. Adiponectin is known to exist in a wide range of multimer complexes in plasma and combines via its collagen domain to create 3 major oligomeric forms: a low-molecular weight (LMW) trimer, a middle-molecular weight (MMW) hexamer, and high-molecular weight (HMW) 12- to 18-mer adiponectin (Kadowaki et al. (2006) Adiponectin and adiponectin receptors in insulin resistance, diabetes, and the metabolic syndrome. J Clin Invest. 116(7): 1784-1792; Rexford S. Ahima, Obesity 2006;14:242S-249S). Adiponectin has been reported to have several physiological actions, such as protective activities against atherosclerosis, improvement of insulin sensitivity, and prevention of hepatic fibrosis.

Adiponectin as used herein, preferably, relates to high molecular weight adiponectin, mid molecular weight adiponectin, more preferably, to total adiponectin, and, most preferably, to low molecular weight adiponectin. The terms high molecular weight adiponectin (12- to 18-mer adiponectin, preferably, 18-mer adiponectin), low and mid molecular weight adiponectin and total adiponectin are understood by the skilled person. Low molecular weigth adiponectin is, preferably, a trimer of three adiponectin polypeptides.

Preferably, the adiponectin is human adiponectin. Methods for the determination of adiponectin are, e.g., disclosed in the Examples or in US 2007/0042424 A1 or WO2008/084003. The adiponectin referred to in accordance with the present invention further encompasses allelic and other variants of said specific sequence for human adiponectin discussed above. Specifically, envisaged are variant polypeptides which are on the amino acid level preferably, at least 50%, 60%, 70%, 80%, 85%, 90%, 92%, 95%, 97%, 98%, or 99% identical to human adiponectin (preferably over the entire length of human adiponectin). The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human adiponectin. Preferably, the adiponectin variants have adiponectin properties, preferably, including its ability to reduce adipose tissue which can be tested in known animal models.

Whether a variant of adiponectin is capable of reducing adipose tissue can be, e.g., tested as follows: The tested variant can be administered to adipocytes, to tissue comprising adipocytes or to an non-human animal. Preferably, a variant of adiponectin is capable of reducing adipose tissue, if the amount of fat comprised by said adipocytes, said tissue or said non-human animal is decreased as compared with control adipocytes, a control tissue or not-human animal which have not been contacted with said variant. Preferably, the non-human animal will be sacrificed after the determining whether said variant reduces adipose tissues (It is, thus, to be understood that such a determination is not deemed to be a method of treatment of the human or animal body).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific detection agent, (b) (optionally) removing non-bound detection agent, (c) measuring the amount of bound detection agent. The bound detection agent will generate an intensity signal. Binding according to the present invention includes both covalent and non-covalent binding. A detection agent according to the present invention can be any compound, e.g., a peptide, polypeptide, nucleic acid, or small molecule, binding to the peptide or polypeptide described herein. Preferred detection agents include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such detection agents are well-known in the art. For example, identification and production of suitable antibodies or aptamers is also offered by commercial suppliers. The person skilled in the art is familiar with methods to develop derivatives of such detection agents with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the detection agent or agent binds specifically to the peptide or polypeptide. Specific binding according to the present invention means that the detection agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the detection agent can be measured by any method known in the art. Preferably, said method is semi-quantitative or quantitative. Further suitable techniques for the determination of a polypeptide or peptide are described in the following.

First, binding of a detection agent may be measured directly, e.g. by NMR or surface plasmon resonance. Second, if the detection agent also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the detection agent may exhibit enzymatic properties itself and the "detection agent/peptide or polypeptide" complex or the detection agent which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label.prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the detection agent may be coupled covalently or non-covalently to a label allowing detection and measurement of the detection agent. Labelling may be done by direct or indirect methods. The detection agent or substrate may also be "tagged" with one or more tags as known in the art. Such tags may then be targets for higher order detection agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immune assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a detection agent for the peptide or polypeptide as specified above with a sample comprising the peptide or polypeptide and (b) measuring the amount peptide or polypeptide which is bound to the support. The detection agent, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The detection agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said detection agent are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different detection agents. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound detection agents. It is to be understood that values correlating to the aforementioned amounts or parameters can also be obtained by all standard mathematical operations.

As set forth above, the sample is, preferably, urine. Preferably, the amount of adiponectin in said urine sample to be tested or in the reference sample is normalized to the amount of creatinine in said urine sample The normalization, preferably, allows to account for creatinine clearance and urine flow.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provides the desired assessment in a suitable output format, i.e. the diagnostic result. The said diagnostic result may, preferably, serve as an aid for establishing the final clinical diagnosis by, e.g., a medical practitioner.

Based on the comparison of the amount determined in step a) and the reference amount, it shall be possible to assess whether a pregnant subject suffers from kidney damage, or not. Therefore, the reference amount is to be chosen so that either a difference or an identity in the compared amounts allows identifying those test subjects which belong into the group of subjects which are either suffering from kidney damage or not. The method allows either excluding (rule-out) or identifying (rule-in) a subject as a subject suffering from kidney damage. Differences in the amounts, i.e. increases or decreases, as used herein, preferably, are differences which are statistically significant. Whether a difference is statistically significant can be determined by the statistical techniques referred to elsewhere herein. Similarly, an identity in the amounts encompasses identical amounts and those differences in the amounts which are not statistically significant and which are within the standard deviations for a measured parameter.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects suffering from kidney damage and subjects not suffering from kidney damage, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject for suffering from kidney damage (i.e. a rule out) whereas an optimal specificity is envisaged for a subject not suffering from kidney damage (i.e. a rule in).

If the subject to be tested is a pregnant, the following applies: Preferably, the reference amount is derived from a pregnant subject or group thereof being at the same stage of gestation as the subject to be tested. More preferably, the reference amount is derived from a pregnant subject being in the same gestational trimester as the pregnant subject to be tested. Even more preferably, the reference amount is derived from a pregnant subject being in the same gestational week as the pregnant subject.

If the subject is tested after pregnancy (in particular after delivery of a child) the following applies with respect to the reference: Preferably, the reference amount is derived from a subject after pregnancy (in particular after delivery of a child) or from a group thereof. Preferably, the sample for the reference amount is obtained not later than one year after delivery. More preferably, said sample is obtained not later than six months, or not later than three months after delivery. Even more preferably, said sample is obtained not later than six weeks, or not later than three after delivery. Most preferably, said sample is obtained not later than 1 week after delivery. Moreover, is particularly envisaged that the test subject as well as the subject from whom the reference amount is derived are in the same week after pregnancy.

The subject (or group thereof) from whom the reference amount is derived, preferably, shall be either known to suffer from kidney damage or shall be known not to suffer from kidney damage.

The following applies as a diagnostic algorithm:
i) an essentially identical amount of adiponectin or an increased amount of adiponectin in the sample of the subject to be tested as compared to the reference amount is indicative for the diagnosis of kidney damage, if the reference amount is derived from a subject suffering from kidney damage (or a group thereof), and/or
ii) an essentially identical amount of adiponectin or a decreased amount of adiponectin in the sample of the subject to be tested as compared to the reference amount indicates that the pregnant subject does not suffer from kidney damage, if the reference amount is derived from a subject not suffering from kidney damage (or from a group thereof).

Preferred reference amounts derived from a subject known to suffer from kidney damage are as follows (in increasing order of preference):
- about 7.5 µg/g, 8.5 µg/g, or 11 µg/g creatinine if the reference amount is derived from a subject being week 9 to 13 of gestation
- about 7.8 µg/g, 8.8 µg/g, or 11.3 µg/g creatinine if the reference amount is derived from a subject being week 14 to 17 of gestation
- about 9.4 µg/g, 10.5 µg/g, or 12.9 µg/g creatinine if the reference amount is derived from a subject being week 18 to 22 of gestation
- about 9.4 µg/g, 10.5 µg/g, or 12.9 µg/g creatinine if the reference amount is derived from a subject being week 23 to 27 of gestation
- about 15 µg/g, 16.5 µg/g, or 20 µg/g creatinine if the reference amount is derived from a subject being week 28 to 32 of gestation
- about 15 µg/g, 16.5 µg/g, or 30 µg/g creatinine if the reference amount is derived from a subject being week 33 to 37 of gestation
- about 25 µg/g, 27 µg/g, or 31 µg/g creatinine if the reference amount is derived from a subject being week 38 or later of gestation
- about 30 µg/g, 35 µg/g, or 40 µg/g creatinine if the reference amount is derived from a subject within three weeks, two weeks, or, more preferably, one week after pregnancy
- about 4 µg/g, 6 µg/g, or 8 µg/g creatinine if the reference amount is derived from a subject being in week 4 to 52, in particular between week 4 to 24 after pregnancy.

Preferred reference amounts derived from a subject known not to suffer from kidney damage are as follows (in increasing order of preference):
- about 4.5 µg/g, 3.5 µg/g, or 2.5 µg/g creatinine if the reference amount is derived from a subject being week 9 to 13 of gestation
- about 4.8 µg/g, 3.8 µg/g, or 1.8 µg/g creatinine if the reference amount is derived from a subject being week 14 to 17 of gestation
- about 6.4 µg/g, 5.4 µg/g, or 4.4 µg/g creatinine if the reference amount is derived from a subject being week 18 to 22 of gestation
- about 6.1 µg/g, 5.1 µg/g, or 4.1 µg/g creatinine if the reference amount is derived from a subject being week 23 to 27 of gestation
- about 13 µg/g, 12 µg/g, or 11 µg/g creatinine if the reference amount is derived from a subject being week 28 to 32 of gestation
- about 13 µg/g, 12 µg/g, or 11 µg/g creatinine if the reference amount is derived from a subject being week 33 to 37 of gestation
- about 21 µg/g, 20 µg/g, or 19 µg/g creatinine if the reference amount is derived from a subject being week 38 or later of gestation
- about 27 µg/g, 26 µg/g, or 25 µg/g creatinine if the reference amount is derived from a subject within three weeks, two weeks, or, more preferably, one week after pregnancy
- about 2 µg/g, 1.5 µg/g, or 1 µg/g creatinine if the reference amount is derived from a subject being in week 4 to 52, in particular between week 4 to 24 after pregnancy.

Preferably, the term "about" as used herein encompasses a range of + and - 20% relative to the specific value, amount, concentration, level, time period etc., e.g., indication of a value of "about 100" is meant to encompass a value of a numerical range of 100 +/- 20%, i.e. a value range from 80 to 120. Preferably, the encompasses a range of + and - 10 % relative to the specific value, amount, concentration, level, time period etc. and, more preferably, a range of + and - 10 % relative to the specific value, amount, concentration, level, time period etc. Most preferably, the term "about" refers to the exact value, amount, concentration, level, etc.

If the subject to be tested suffers from diabetes type 1, diabetes type 2 and pre-eclampsia, respectively, the subject from whom the reference amount is derived shall preferably also suffer from the same condition, i.e. from diabetes type 1, diabetes type 2 and preeclampsia, respectively.

Advantageously, it has been shown in the context of the present invention that adiponectin is a reliable marker for kidney disease in pregnant women or in women after delivery, whereas other markers/indicators for kidney damage such as serum creatinine, U-LABP, KIM-1 and the GFR proved to be less reliable (see Williams et al., loc. cit, or Examples). In particular, it has been shown that adiponectin levels are generally increased during and after pregnancy. Therefore, a reference amount that has not been obtained from a woman during or after pregnancy is not applicable for carrying out the method according to the present invention. Accordingly, the reference amount for carrying out the diagnosis has to be derived from a pregnant woman or from a woman after delivery (of from a group thereof) since the use of other reference amounts would lead to a large number of false positive test results.

It is to be understood that the definitions and explanations of the terms made above and below apply accordingly for all embodiments described in this specification and the accompanying claims.

In a preferred embodiment of the method of the present invention, said method further comprises the step of recommending a therapeutic measure if kidney damage has been diagnosed. Preferably, said therapeutic measure shall aim to treat kidney damage.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention. The recommendation step referred to above can also, preferably, be automated. Preferably, the diagnosis or aid for diagnosis obtained from the step b) of the method of the present invention, i.e. the diagnostic result of the method, will be used to search a database comprising recommendations of therapeutic measures for the individual possible diagnostic results.

Also, the present invention relates to the use of i) the biomarker adiponectin or ii) to the use of a detection agent which specifically binds to adiponectin in sample of a subject for diagnosing kidney damage in a subject during or after pregnancy.

The term "detection agent" as used herein refers to an agent which is capable of specifically recognizing and binding to adiponectin present in a sample. The agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker protein or a nucleic acid encoding the biomarker. The term "antibody" as used herein includes both polyclonal and monoclonal antibodies, as well as any modifications or fragments thereof, such as Fv, Fab and F(ab)₂ fragments. The antibody shall be capable of specifically binding to adiponectin.

Moreover, the present invention relates to a device adapted for carrying out the method of the present invention for diagnosing kidney damage during or after pregnancy, comprising
a. an analyzing unit comprising a detection agent which specifically binds to adiponectin, said unit being adapted for determining the amount of adiponectin in a sample of a subject; and
b. an evaluation unit for comparing the determined amount with a reference amount whereby kidney damage can be diagnosed, said unit comprising a database with a value for a reference amount (or values for reference amounts) for adiponectin and a computer-implemented algorithm for carrying out a comparison step.

Preferred reference amounts are disclosed herein elsewhere. Preferably, the reference amount is obtained from a subject during or after pregnancy as described elsewhere herein.

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference (e.g. a reference amount, or the amount of the marker in a first or second sample from the subject). Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician.

Moreover, the present invention envisages a kit adapted for carrying out the method of the present invention for diagnosing kidney disease in a subject during or after pregnancy, said kit comprising a detection agent for the biomarker adiponectin. Moreover, the kit further may comprise instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards for reference amounts as described elsewhere herein in detail.

All references cited in this specification are herewith incorporated by reference with respect to their entire disclosure content and the disclosure content specifically mentioned in this specification.

### EXAMPLES

### Example 1: Determination of the biomarkers adiponectin, KIM-1 (Kidney injury marker 1) and L-FABP (Liver fatty acid binding protein)

Adiponectin, KIM-1 (Kidney injury marker 1) and L-FABP (Liver fatty acid binding protein) were determined in urine samples. The samples were kept at least at -20°C until tested.

Adiponectin (multimeric) was determined by using the test EIA from ALPCO diagnostics (USA), operating on the principle of a "sandwich" format ELISA. The specific antibodies used in the kit are anti-human adiponectin monoclonal antibodies (MoAbs) directed to two independent epitopes. The specimens were pre-treated as described below, and total adiponectin and individual multimers of adiponectin are determined selectively, directly or indirectly. Multimers of adiponectin are classified into four fractions with this kit:
1) Total adiponectin fraction: "Total-Ad"-assayed directly on the plate
2) High-molecular adiponectin fraction (equivalent of dodecamer-octodecamer):
   "HMW-Ad"-assayed directly on the plate
3) Middle-molecular adiponectin fraction (equivalent of hexamer): "MMW-Ad"-inferred value obtained by subtracting the concentration of HMW-Ad from the combined concentration of MMW-Ad + HMW-Ad
4) Low-molecular adiponectin fraction (equivalent of trimer including albuminbinding adiponectin): "LMWAd"-inferred value obtained by subtracting the combined concentration of MMW-Ad + HMW-Ad from the total concentration of Ad. The microtiter plate wells have been coated with an anti-human adiponectin monoclonal antibody. Adiponectin in the standards and pretreated specimens are captured by the antibody during the first incubation. Afterwards, a wash step removes all unbound material. Subsequently, an anti-human adiponectin antibody which has been biotin-labeled is added and binds to the immobilized adiponectin in the wells. Subsequently, an anti-human adiponectin antibody which has been biotin-labeled is added and binds to the immobilized adiponectin in the wells. After the second incubation and subsequent was step, HRP-labeled streptavidin is added. After the third incubation and subsequent was step, substrate solution is added. Finally, stop reagent is added after allowing the color to develop. The intensity of the color development is read by a microplate reader. The absorbance value reported by the plate reader is proportional to the concentration of adiponectin in the sample.

U-LFABP was determined by using the L-FABP ELISA kit from CMIC, Ltd, Japan. The test is based on an ELISA 2 step assay. L-FABP standard or urine samples are firstly treated with pre-treatment solution provided by the manufacturer, and transferred into a L-FABP antibody coated microplate containing assay buffer and incubated. During this incubation, L-FABP in the reaction solution binds to the immobilized antibody. After washing, the 2^{nd} antibody-peroxidase conjugate is added as the secondary antibody and incubated, thereby forming sandwich of the L-FABP antigen between the immobilized antibody and conjugate antibody. After incubation, the plate is washed and substrate for enzyme reaction is added, color develops according to the L-FABP antigen quantity. The L-FABP concentration is determined based on the optical density. The measurement range of the kit is 3 to 400 ng/ml.

Human KIM-1 was determined in urine samples by the human KIM-1 (catalogue number DY 1750) ELISA development kit from R&D systems, containing a capture antibody (goat anti-human TIM-1) and a detection antibody (biotinylated goat anti-human TIM 1). A seven point standard curve using 2 fold serial dilutions in reagent diluent provided by the manufacturer, and a high standard of 2000 pg/ml is recommended.

The measured amounts of the biomarkers were normalized to the amount of creatinine in the urine sample.

### Example 2: Patient cohorts

Pregnant women: Urine samples were available from 45 pregnant women with uneventful pregnancy, 8 pregnant women had diabetes mellitus which was known to be present before they became pregnant (pregestional diabetes mellitus), 12 pregnant women were diagnosed with gestational diabetes mellitus and 17 pregnant women met the criteria of preeclampsia. All pregnant women had normal kidney function and thus no evidence of chronic kidney disease. In some but not all pregnant subjects follow up samples during gestation and thereafter were available.

Several control groups as indicated herein below were also analyzed. Patients in all control groups had normal kidney function as assessed by creatinine levels within the normal range. None of the control patients/subjects was pregnant at the time of testing or within the previous year.

Normal subjects: Urine sample were taken from 149 clinically healthy subjects (52 males and 97 females (median age 41 years, range: 19 to 56 years). The subjects has no diabetes mellitus, no known urinary tract infections in addition they had no cardiac diseases as assessed by medical history and an electrocardiogram.

Patients with heart failure: 42 patients with established heart failure were included into the study. All patients were clinically stable indicated by lack of acute symptoms for the preceeding month, stable cardiac medication and change in body weight below 2 kg within the past month. Median age of the patients was 62 years (43-71 years), they were 31 males and 10 females. All patients had a left ventricular ejection fraction below 40 % and thus systolic dysfunction. All patients had ischemic heart disease indicating that coronary artery disease was the assumed cause of heart failure.

Patients with type 1 diabetes mellitus: A total of 203 patients with type 1 diabetes mellitus were included into the study (median age 41 years, range 27 - 57 years), 132 males and 71 females). None of the patients had apparent cardiac disease or acute urinary tract infection at the time of sampling or in the preceding 4 weeks.

Patients with type 2 diabetes mellitus: A total of 134 patients with type 2 diabetes mellitus were included into the study (median age 56 years (range 49 - 69 years), they were 78 males and 56 females. All patients lacked apparent cardiac disease and they did not have urinary tract infection within 4 weeks before sampling of urine.

### Example 3: Median levels of the markers in the analyzed cohorts

Table 1 gives an overview on the median levels of the various biomarkers in the analyzed cohorts. The levels are given in µg/g creatinine for total molecular weight adiponectin, in µg/g creatinine for KIM-1, and in µg/g creatinine for L-FABP.

**Table 1**

| Median levels in non-pregnant women | | | |
|---|---|---|---|
| | Adiponectin | U LFABP | KIM 1 |
| Normal subjects | 0,66 | 3,78 | 0,33 |
| | | | |
| D. mellitus 2 | 0,4 | 5,5 | 0,3 |
| D mellitus 1 | 0,33 | 8,21 | 0,38 |
| Heart failure | 0,14 | 7,7 | 0,57 |
| | | | |

| Median levels during pregnancy | | | |
|---|---|---|---|
| Normal pregnancy | 5,2 | 8,0 | 0,44 |
| | | | |

| Abnormal pregnancy | | | |
|---|---|---|---|
| Gestational D. mell. | 2,6 | 4,2 | 1,3 |
| D. mellitus | 9,0 | 8,8 | 0,6 |
| Pre-eclampsia (PE) | 8,9 | 6,8 | 0,8 |
| | | | |

| Median levels 24 weeks after deliverv (24 weeks pp: post partum) | | | |
|---|---|---|---|
| normal pregnancy | 1,9 | 6,4 | 0,7 |
| abnormal pregnancy | 3,9 | 7,0 | 0,8 |

The amounts of adiponectin in pregnant women are higher than in non pregnant women, this is even the case 24 weeks after delivery which is of importance for the identification of pathological conditions. Pathological conditions are preferably identified by adiponectin. Pathological conditions as indicated by the increase of adiponectin were present in D. mellitus and in preeclampsia. To a much lesser extent than by adiponectin, pathological conditions can be identified by KIM-1. No conclusions can be drawn by using L-FABP as a marker. The amount of L-FABP in normal pregnancy is even larger than the amount of L-FABP in gestational diabetes and pre-eclampsia (PE). The results are surprising since KIM-1 and L-FABP are usually good markers for kidney damage. However, the results of the present study show that this is not the case for pregnant women.

Increased markers of adiponectin have been proposed as a marker for vascular damage in type 2 diabetes (see von Eynatten, 2009, Diabetes, 58: 2093 to 2099). Interestingly, increased levels of adiponectin observed in normal pregnancy usually do not indicate kidney damage and, thus, do not correlate with pathological conditions. Therefore, the reference amount to be used in the context of the present invention has to be derived from a pregnant woman in order to allow for a reliable diagnosis. If other reference amounts would be used such as reference amounts derived from a non-pregnant woman, the diagnosis would be less reliable since almost any pregnant women would be considered to exhibit kidney damage. Thus, the use of other reference amounts would result in a large number of wrong positive results.

### Example 4: Relationship between the measured biomarkers and the gestational age

Median levels of kidney markers related to gestational age are shown in Table 2.

**Table 2**

| | Adiponectin | U LFABP | KIM 1 |
|---|---|---|---|
| 9 - 13 | 4,5 | 34 | 0,7 |
| 14 - 17 | 4,8 | 11 | 0,5 |
| 18 - 22 | 6,4 | 7 | 0,6 |
| 23 - 27 | 6,1 | 5 | 1,0 |
| 28 - 32 | 12,8 | 6 | 0,6 |
| 33 - 37 | 11,9 | 11 | 0,9 |
| 38 - p | 21,0 | 14 | 1,4 |
| 1 week pp | 27 | 37 | 1,6 |
| 6 weeks pp | 1,9 | 6 | 0,6 |
| 24 weeks pp | 1,8 | 6 | 0,7 |

As it can be seen from the table, the amount of adiponectin increases during pregnancy are remains elevated in the first weeks after delivery. Therefore, the reference amount for carrying out the method of the present invention is, preferably, derived from a woman being in the same gestational stage or from a woman after delivery.

### Example 5: Case studies

Case 1: A 32 year old female with long standing type 1 diabetes mellitus in whom pregnancy was excluded has an adiponectin level of 1,2 ug/g creatinine. Glucose and HbAlc levels are slightly elevated. The patient is diagnosed with diabetes associated glomerular damage and recommended to improve diet and glucose profile and was suggested to take ACE inhibitors.

Case 2: A 29 old pregnant female with well controlled diabetes mellitus who is in the 33rd gestatinonal week has a serum adiponcetin level in urine of 9,8 ug/g creatinine. She is informed that her diabetes is well controlled. The increased adiponectin levels are within the normal range according to the gestational age and do not indicate kidney disease. No further dietary of therapeutic advice is needed.

Case 3: A 28 year old pregnant female (31st week of gestation) without any previous disease has an adiponectine levels in urine of 21.3 ug/g creatinine. The elevated creatinine levels is clearly abnormal even in pregnancy. A ultrasound of the kidney is within normal range, so is kidney function according to creatinine levels in serum. Diabetes mellitus is also excluded. She is suspected to suffer from glomerulonephritis. Because of the existing pregnancy she is advised to return for regular follow up.

Case 4: A 36 year old female who had delivered 6 months ago presents to the nephrologists, her gynecologist as recognized adiponectin concentrations of 1.5 ug/g creatine in her urine which were far above concentration in healthy females. She is informed that the amounts of adiponectin during or after pregnancy are not comparable to those who are not pregnant and that increased adiponectin levels as compared to non-pregant women may persist up to 1 year after delivery. Her adiponectin value is considered to be normal in this context and no further diagnostic procedures are needed at that time. A follow up in 1 year is recommended.

### Conclusion:

Pregnancy is associated with substantial hormonal changes that affect different organs and specifically the kidney. In the context of the present invention it was found that reference values for adiponectin for the diagnosis of kidney disease in non-pregnant women do apply to pregnant women and also not to women who have delivered in the past year. Thanks to the present invention using appropriate reference value kidney disease(preferably glomerular kidney disease) can be diagnosed or excluded which would have been otherwise misdiagnosed.

## Claims

1. A method for diagnosing kidney damage in a subject during or after pregnancy, comprising the steps of
a. determining the amount of adiponectin in a sample from said subject
b. comparing the amount of adiponectin in said sample from said subject with a reference amount for adiponectin, said reference amount being derived from a pregnant subject or from a subject after pregnancy,
whereby kidney damage is to be diagnosed.

2. The method of claim 1, wherein said kidney damage is glomerular damage.

3. The method of claim 1 or 2, wherein kidney damage in a subject during pregnancy is diagnosed, and wherein the reference amount is derived from a pregnant subject being in the same gestational stage as the subject to be tested.

4. The method of claim 3, wherein the reference amount is derived from a pregnant subject being in the same gestational week as the subject to be tested.

5. The method of claim 1 or 2, wherein kidney damage in a subject after pregnancy is diagnosed, and wherein the reference amount is derived from a subject after pregnancy.

6. The method of claim 5, wherein the reference amount the reference amount is derived from a subject not later than 6 months after delivery of a child.

7. The method of any one claims 1 to 6, wherein the subject from whom the reference amount is derived suffers from kidney damage, and wherein an essentially identical amount of adiponectin or an increased amount of adiponectin in the sample of the subject to be tested as compared to the reference amount is indicative for the diagnosis of kidney damage.

8. The method of claims 1 to 6, wherein the subject from whom the reference amount is derived does not suffer from kidney damage, and wherein an essentially identical amount of adiponectin or a decreased amount of adiponectin in the sample of the subject to be tested as compared to the reference amount indicates that the subject does not suffer from kidney damage.

9. The method of any one of claims 1 to 8, wherein the sample is urine.

10. The method of any one of claims 1 to 9, wherein the subject is human.

11. Use of the biomarker adiponectin, or of a detection agent which specifically binds to adiponectin in sample of a subject for diagnosing kidney damage in a subject during or after pregnancy.

12. A device adapted for carrying out the method of any one of claims 1 to 10, comprising
a. an analyzing unit comprising a detection agent which specifically binds to adiponectin, said unit being adapted for determining the amount of adiponectin in a sample of a subject; and
b. an evaluation unit for comparing the determined amount with a reference amount whereby kidney damage can be diagnosed, said unit comprising a database with a value for a reference amount as defined in any of claims 3 to 6, and a computer-implemented algorithm for carrying out a comparison step.
